(19) Europäisches Patentamt
European Patent Office
Office européen des brevets

(11) Veröffentlichungsnummer: 0 207 891
A1

(12) EUROPÄISCHE PATENTANMELDUNG

(21) Anmeldenummer: 86810256.7

(22) Anmeldetag: 09.06.86

(51) Int.Cl.⁴: C 07 D 275/06
A 01 N 43/80

(30) Priorität: 14.06.85 CH 2532/85
05.05.86 CH 1824/86

(43) Veröffentlichungstag der Anmeldung:
07.01.87 Patentblatt 87/2

(84) Benannte Vertragsstaaten:
AT BE CH DE FR GB IT LI NL

(71) Anmelder: CIBA-GEIGY AG
Klybeckstrasse 141
CH-4002 Basel(CH)

(72) Erfinder: Drabek, Jozef, Dr.
Benkenstrasse 12
CH-4104 Oberwil(CH)

(72) Erfinder: Fürer, Richard
Feldstrasse 21
CH-4123 Allschwil(CH)

(54) **Salze von 3-Acylaminobenzisothiazol-S,S-dioxiden, Verfahren zu ihrer Herstellung und ihre Verwendung in der Schädlingsbekämpfung.**

(57) Salze von 3-Acylaminobenzisothiazol-S, S-dioxiden der Formel

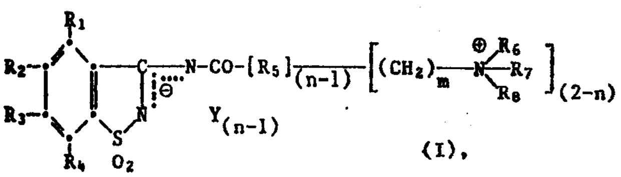

(I),

worin

$R_1$, $R_2$, $R_3$ und $R_4$ unabhängig voneinander Wasserstoff, Halogen, $C_1$-$C_5$-Alkyl, $C_1$-$C_5$-Alkoxy, Trifluormethyl, Amino oder Nitro,

Y $Na^{\oplus}$, $K^{\oplus}$ oder

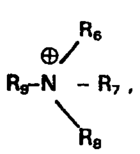

$R_5$ gegebenenfalls substituiertes Alkyl, Cycloalkyl, Alkenyl, Alkinyl oder Phenyl,

$R_6$, $R_7$ und $R_8$ unabhängig voneinander Alkyl, Alkenyl oder Alkinyl,

$R_9$ Wasserstoff, Alkyl, Alkenyl oder Alkinyl,

n die Zahl 1 oder 2 und

m eine Zahl von 1 bis 10 bedeuten.

Es werden Verfahren zur Herstellung dieser Verbindungen und ihre Verwendung in der Schädlingsbekämpfung beschrieben.

EP 0 207 891 A1

0207891

5-15385/1+2

# Salze von 3-Acylaminobenzisothiazol-S,S-dioxiden, Verfahren zu ihrer Herstellung und ihre Verwendung in der Schädlingsbekämpfung

Die vorliegende Erfindung betrifft Salze von 3-Acylaminobenzisothiazol-S,S-dioxiden, Verfahren zu ihrer Herstellung und ihre Verwendung in der Schädlingsbekämpfung. Die Salze von 3-Acylaminobenzisothiazol-S,S-dioxiden haben die Formel

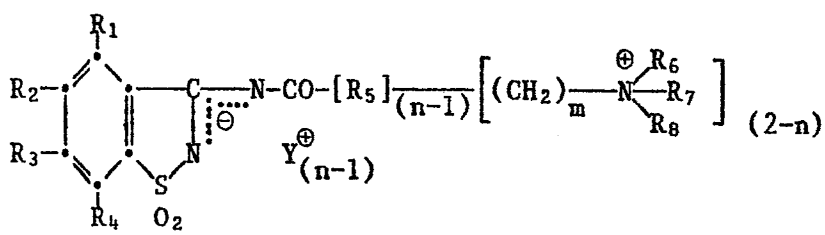

(I),

worin

$R_1$, $R_2$, $R_3$ und $R_4$ unabhängig voneinander Wasserstoff, Halogen, $C_1$-$C_5$-Alkyl, $C_1$-$C_5$-Alkoxy, Trifluormethyl, Amino oder Nitro,

Y $Na^{\oplus}$, $K^{\oplus}$ oder 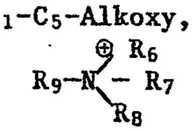 ,

$R_5$ gegebenenfalls substituiertes Alkyl, Cycloalkyl, Alkenyl, Alkinyl oder Phenyl,

$R_6$, $R_7$ und $R_8$ unabhängig voneinander Alkyl, Alkenyl oder Alkinyl,

$R_9$ Wasserstoff, Alkyl, Alkenyl oder Alkinyl,

n die Zahl 1 oder 2 und

m eine Zahl von 1 bis 10 bedeuten.

Halogen kann dabei für Fluor, Chlor, Brom oder Jod, insbesondere aber für Chlor oder Fluor, stehen.

Die bei $R_1$ bis $R_8$ in Frage kommenden Alkyl-, Alkoxy-, Alkenyl- und Alkinylgruppen können geradkettig oder verzweigt sein. Die Alkyl-, Alkenyl- und Alkinylgruppen bei $R_5$ - $R_9$ haben 1 bis 10 resp. 2 bis 10, bevorzugt 1 bis 5 resp. 2 bis 5, Kohlenstoffatome.

Die Cycloalkylgruppen bei $R_5$ haben 3 bis 7, bevorzugt 3 bis 5, Kohlenstoffatome.

Beispiele von Substituenten an den Alkyl-, Alkenyl, Alkinyl-, Cycloalkyl- und Phenylgruppen bei $R_5$ sind u.a.: Halogen, $C_1$-$C_5$-Alkoxy, Amino und/oder Trifluormethyl. Die Cycloalkyl- und Phenylgruppen bei $R_5$ können auch durch $C_1$-$C_5$-Alkyl oder $C_1$-$C_5$-Halogenalkyl substituiert sein.

Beispiele von Alkyl-, Alkoxy-, Cycloalkyl-, Alkenyl- oder Alkinylgruppen bei $R_1$ bis $R_9$ sind u.a.: Methyl, Methoxy, $-CH_2Cl$, Trifluormethyl, Aethyl, Aethoxy, $-CH_2CH_2F$, $-CF_2CF_3$, Propyl, $-CF_2CF_2CF_3$, Isopropyl, n-, i-, sek.-, tert.-Butyl, n-Pentyl, n-Hexyl, n-Decyl und deren Isomere, 2-Propenyl, 2-Propinyl, Cyclopropyl, Cyclopentyl.

Besonders erwähnt seien die Verbindungen der Formel I, in denen $R_1$, $R_2$, $R_3$ und $R_4$ unabhängig voneinander Wasserstoff, Halogen, $C_1$-$C_5$-Alkyl, $C_1$-$C_5$-Alkoxy oder Trifluormethyl,

Y $Na^{\oplus}$, $K^{\oplus}$ oder $\overset{\oplus}{H}N(R_6)(R_7)(R_8)$ ,

$R_5$ gegebenenfalls durch Halogen oder $C_1$-$C_5$-Alkoxy substituiertes $C_1$-$C_{10}$-Alkyl, $C_2$-$C_{10}$-Alkenyl oder $C_2$-$C_{10}$-Alkinyl oder gegebenenfalls auch durch Halogen, $C_1$-$C_5$-Alkyl, $C_1$-$C_5$-Halogenalkyl oder $C_1$-$C_5$-Alkoxy substituiertes $C_3$-$C_7$-Cycloalkyl oder Phenyl,

$R_6$, $R_7$ und $R_8$ unabhängig voneinander $C_1$-$C_{10}$-Alkyl, $C_2$-$C_{10}$-Alkenyl oder $C_2$-$C_{10}$-Alkinyl,

n die Zahl 1 oder 2 und

m die Zahl 1 bedeuten.

Unter den Verbindungen der Formel I stehen diejenigen im Vordergrund, in denen $R_1$ Halogen, $R_2$, $R_3$ und $R_4$ Wasserstoff, Y $Na^{\oplus}$, $K^{\oplus}$ oder 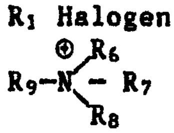 ,
$R_5$ $C_1$-$C_5$-Alkyl, $R_6$, $R_7$, $R_8$ und $R_9$ unabhängig voneinander $C_1$-$C_4$-Alkyl, und n die Zahl 2 bedeuten.

Dabei sind diejenigen Verbindungen der Formel I bevorzugt, in denen $R_1$ Chlor, $R_2$, $R_3$ und $R_4$ Wasserstoff, Y $Na^{\oplus}$ oder $K^{\oplus}$, $R_5$ Methyl, und n die Zahl 2 bedeuten.

Die Verbindungen der Formel I werden nach an sich bekannten Methoden z.B. wie folgt hergestellt:

1) 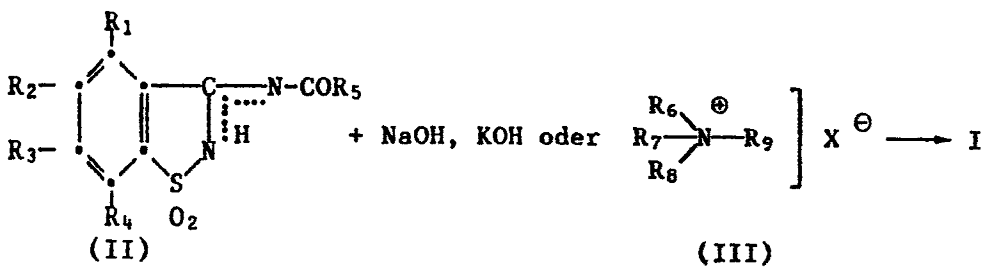

(II) (III)

2) 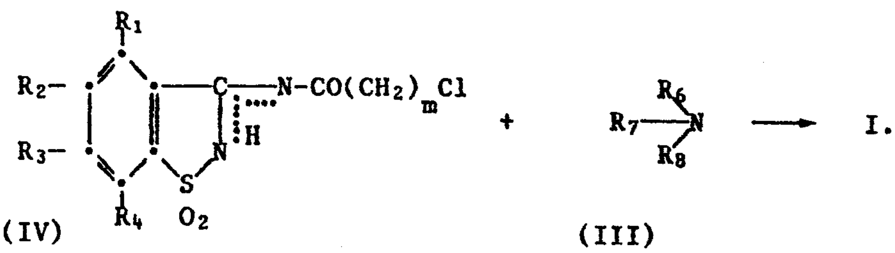
(IV) (III)

In den Formeln II, III und IV haben $R_1$ - $R_9$ und m die für die Formel I angegebene Bedeutung und X steht für ein Hydroxyl- oder Halogenanion. Die Verfahren werden im Vakuum oder bei normalem Druck bei einer Temperatur von 0° bis 100°C, vorzugsweise bei 20° bis 50°C, und gegebenenfalls in einem Lösungs- oder Verdünnungsmittel durchgeführt.

Als Lösungs- oder Verdünnungsmittel eignen sich z.B. Aether und ätherartige Verbindungen, wie Diäthyläther, Diisopropyläther, Dioxan, 1,2-Dimethoxyäthan und Tetrahydrofuran; Amide, wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Verbindungen der Formeln II, III und IV sind bekannt oder können analog bekannten Methoden hergestellt werden.

Die Verbindungen der Formel I eignen sich zur Bekämpfung von verschiedenartigen Schädlingen an Tieren und Pflanzen sowie im Boden. So können sie zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Acarina eingesetzt werden.

So eignen sich Verbindungen der Formel I zur Bekämpfung von pflanzenschädigenden Insekten in Zier- und Nutzpflanzen, insbesondere in Baumwollkulturen (z.B. Spodoptera littoralis und Heliothis virescens). Vor allem aber haben Verbindungen der Formel I eine günstige Wirkung gegen Bodeninsekten (z.B. Aulacophora femoralis, Chortophila brassicae, Diabrotica balteata, Pachnoda savignyi und Scotia ypsilon).

Wirkstoffe der Formel I zeigen auch eine sehr günstige Wirkung gegen Fliegen, wie z.B. Musca domestica, sowie Mückenlarven. Ferner zeichnen sie sich durch eine breite ovizide und ovolarvizide Wirkung aus und besitzen eine wertvolle Wirkung gegen ektoparasitäre Milben und Zecken z.B. der Familien Ixodidae, Argasidae und Dermanyssidae. Ausserdem besitzen die Verbindungen der Formel I auch gute nematizide Eigenschaften.

Die gute pestizide Wirkung der erfindungsgemässen Verbindungen der Formel I entspricht einer Abtötungsrate (Mortalität) von mindestens 50-60 % der erwähnten Schädlinge.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; pyrethrinartige Verbindungen sowie Karbamate und chlorierte Kohlenwasserstoffe.

Die Verbindungen der Formel I wirken auch gegen phytopathogene Pilze. So wirken Verbindungen der Formel I gegen die den folgenden Klassen angehörenden phytopathogenen Pilze: Ascomycetes (z.B. Erysiphaceae, Fusarium, Helminthosporium); Basidiomycetes (wie z.B. Puccinia, Rhizoctonia, Tilletia, Hemileia); Fungi imperfecti (z.B. Cercospora, Botrytis, Septoria); Phycomycetes (wie z.B. Phytophthora).

Die Verbindungen der Formel I können vorteilhaft als Beizmittel zur Behandlung von Saatgut und Vorrat (Früchte, Knollen, Körner) und Pflanzenstecklingen und zur Saatfurchenapplikation zum Schutz vor Pilzinfektionen und Insekten sowie gegen die im Erdboden auftretenden phytopathogenen Pilze und Insekten eingesetzt werden.

Mit besonderem Vorteil werden Verbindungen der Formel I auch mit Substanzen kombiniert, welche einen synergistischen oder verstärkenden Effekt ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die mindestens einen Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Essigester, Propylmyristat oder Propylpalmitat, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; Silikonöle oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B.

die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5-Aethylenglykoleinheiten.

Als Beispiele nichtionischer Tenside seien Nonylphenolpolyäthoxyäthanole, Ricinusölpolyglycoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als

Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"McCutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1:

A) Herstellung von Natrium-3-acetylamino-4-chlor-benziso-thiazol-S,S-dioxid

---

5,15 g 3-Acetylamino-4-chlor-benzisotiazol-S,S-dioxid werden in einer Lösung von 0,81 g NaOH in 30 ml Wasser gelöst. Die Lösung wird dann im Vakuum bei max. 50°C eingeengt. Man erhält die Verbindung der Formel

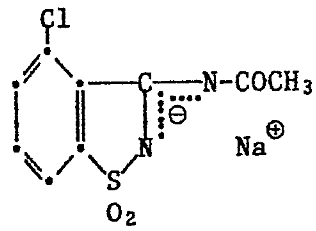

(Verb. 1)

als weisses Pulver mit einem Schmelzpunkt von 260-265°C.

Auf analoge Weise werden auch folgende Verbindungen hergestellt.

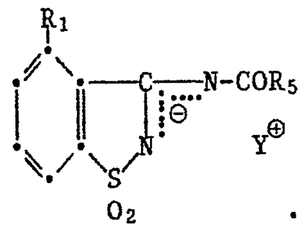

| Verbindung No. | $R_1$ | $R_5$ | $Y^{\oplus}$ | Smp. °C |
|---|---|---|---|---|
| 2 | Cl | $-(CH_2)_4CH_3$ | Na | Smp. 230/Zers. |
| 3 | Cl | $CH_3$ | $HN(C_2H_5)_3$ | Smp. 203-206 |
| 4 | Cl | $CH_3$ | $N(C_2H_5)_4$ | Smp. 125-131 |
| 5 | Cl | $CH_3$ | $N(C_4H_9)_4$ | Smp. 112 |
| 6 | Cl | $CH_3$ | $N(CH_3)_3$ | Smp. 170-172 |
| 7 | Cl | $CH_3$ | K | Smp. 165/Zers. |
| 8 | F | $CH_3$ | Na | Smp. > 265 |

B) Herstellung von 3-Trimethylammoniumacetylamino-4-chlor-benzisothiazol-S,S-dioxid

Zu einer Lösung von 7,32 g 3-Chloracetylamino-4-chlor-benzisothiazol-S,S-dioxid in 150 ml Acetonitril werden bei 20°C 1,8 g Triäthylamin, gelöst in 20 ml Acetonitril, zugetropft. Das Reaktionsgemisch wird dann 5 Stunden bei 20°C gerührt. Die ausgefallene Substanz wird abgenutscht, auf der Nutsche mit Acetonitril gewaschen und im Vakuum getrocknet. Man erhält die Verbindung der Formel

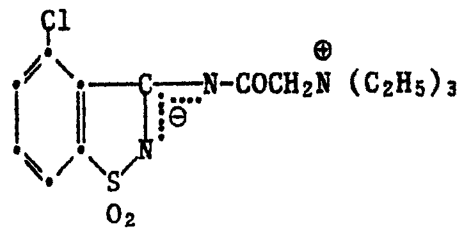

(Verb. 9)

mit einem Schmelzpunkt von 257°C/Zers.

Beispiel 2: Formulierungsbeispiele für Wirkstoffe gemäss dem Herstellungsbeispiel 1

(% = Gewichtsprozent)

| 2.1 Emulsions-Konzentrate | a) | b) | c) | d) |
|---|---|---|---|---|
| Wirkstoff gemäss dem Herstellungsbeispiel 1 | 10 % | 25 % | 40 % | 50 % |
| Ca-Dodecylbenzolsulfonat | - | 5 % | 8 % | 6 % |
| Ricinusöl-polyäthylenglykoläther (36 Mol AeO) | - | 5 % | - | - |
| Tributylphenol-polyäthylenglykoläther (30 Mol AeO) | - | - | 12 % | 4 % |
| Ricinusölthioxilat | 25 % | - | - | - |
| Cyclohexanon | - | - | 15 % | 20 % |
| Butanol | 15 % | - | - | - |
| Xylolgemisch | - | 65 % | 25 % | 20 % |
| Essigester | 50 % | - | - | - |

Aus solchen Konzentraten können durch Verdünnen mit Wasser Emulsionen jeder gewünschten Konzentration hergestellt werden.

| 2.2 Lösungen | a) | b) |
|---|---|---|
| Wirkstoff gemäss dem Herstellungsbeispiel 1 | 10 % | 5 % |
| Aethylenglykol-monomethyläther | - | - |
| Polyäthylenglykol (MG 400) | 70 % | - |
| N-Methyl-2-pyrrolidon | 20 % | - |
| Epoxidiertes Kokosnussöl | - | 1 % |
| Benzin (Siedegrenzen 160-190°C) | - | 94 % |

Die Lösungen sind zur Anwendung in Form kleinster Tropfen geeignet.

| 2.3 Granulate | a) | b) |
|---|---|---|
| Wirkstoff gemäss dem Herstellungsbeispiel 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) | c) | d) |
|---|---|---|---|---|
| Wirkstoff gemäss dem Herstellungsbeispiel 1 | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff erhält man gebrauchsfertige Stäubemittel.

2.5 Spritzpulver

| | a) | b) | c) |
|---|---|---|---|
| Wirkstoff gemäss dem Herstellungsbeispiel 1 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6 Extruder Granulat

| | |
|---|---|
| Wirkstoff gemäss dem Herstellungsbeispiel 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.7 Umhüllungs-Granulat

| | |
|---|---|
| Wirkstoff gemäss dem Herstellungsbeispiel 1 | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

2.8 Suspensions-Konzentrat

| | |
|---|---|
| Wirkstoff gemäss dem Herstellungsbeispiel 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologiebeispiele für Wirkstoffe gemäss dem Herstellungsbeispiel 1

3.1 Wirkung gegen Lucilia sericata

Zu 9 ml eines Zuchtmediums wird bei 50°C ein ml einer 0,1 % Aktivsubstanz enthaltenden wässrigen Zubereitung hinzugefügt. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt. Verbindungen gemäss Beispiel 1 zeigen in diesem Test gute Wirkung gegen Lucilia sericata.

3.2 Wirkung gegen Aëdes aegypti

Auf die Oberfläche von 150 ml Wasser, das sich in einem Behälter befindet, wird so viel einer 0,1%igen acetonischen Lösung des Wirkstoffs pipettiert, dass eine Konzentration von 12,5 ppm erhalten wird. Nach Verdunsten des Acetons wird der Behälter mit 30 bis 40 2tägigen Aëdes-Larven beschickt. Nach 2 und 7 Tagen wird die % Mortalität (Anzahl der schwimmunfähigen Larven) geprüft.

Verbindungen gemäss Beispiel 1 zeigen gute Wirkung (Mortalität) im obigen Test.

3.3 Insektizide Kontaktwirkung: Myzus persicae

Etwa 4 cm hohe, in Wasser angezogene Erbsenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 400 ppm der zu prüfenden Verbindung bis zur Tropfnässe besprüht. Man verwendet pro Konzentration zwei Pflanzen. Eine Auswertung der erzielten Abtötungsrate erfolgt 48 Stunden nach Applikation. Der Versuch wird bei 20-22°C und 60 % relativer Luftfeuchtigkeit durchgeführt.

Verbindungen der Formel I gemäss Beispiel 1 zeigen in diesem Test eine gute Wirkung.

Patentansprüche

1. Ein Salz von 3-Acyl-aminobenzisothiazol-S,S-dioxiden der Formel

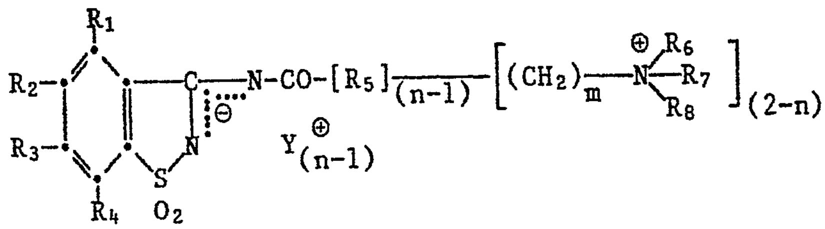 (I),

worin

$R_1$, $R_2$, $R_3$ und $R_4$ unabhängig voneinander Wasserstoff, Halogen, $C_1$-$C_5$-Alkyl, $C_1$-$C_5$-Alkoxy, Trifluormethyl, Amino oder Nitro,

Y $Na^{\oplus}$, $K^{\oplus}$ oder 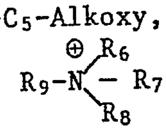 ,

$R_5$ gegebenenfalls substituiertes Alkyl, Cycloalkyl, Alkenyl, Alkinyl oder Phenyl,

$R_6$, $R_7$ und $R_8$ unabhängig voneinander Alkyl, Alkenyl oder Alkinyl,

$R_9$ Wasserstoff, Alkyl, Alkenyl oder Alkinyl,

n die Zahl 1 oder 2 und

m eine Zahl von 1 bis 10 bedeuten.

2. Eine Verbindung gemäss Anspruch 1, worin

$R_1$, $R_2$, $R_3$ und $R_4$ unabhängig voneinander Wasserstoff, Halogen, $C_1$-$C_5$-Alkyl, $C_1$-$C_5$-Alkoxy oder Trifluormethyl,

Y $Na^{\oplus}$, $K^{\oplus}$ oder 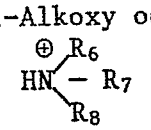 ,

$R_5$ gegebenenfalls durch Halogen oder $C_1$-$C_5$-Alkoxy substituiertes $C_1$-$C_{10}$-Alkyl, $C_2$-$C_{10}$-Alkenyl oder $C_2$-$C_{10}$-Alkinyl oder gegebenenfalls auch durch Halogen, $C_1$-$C_5$-Alkyl, $C_1$-$C_5$-Halogenalkyl oder $C_1$-$C_5$-Alkoxy substituiertes $C_3$-$C_7$-Cycloalkyl oder Phenyl,

$R_6$, $R_7$ und $R_8$ unabhängig voneinander $C_1$-$C_{10}$-Alkyl, $C_2$-$C_{10}$-Alkenyl oder $C_2$-$C_{10}$-Alkinyl,

n die Zahl 1 oder 2 und

m die Zahl 1 bedeuten.

3. Eine Verbindung gemäss Anspruch 1, worin

$R_1$ Halogen,

$R_2$, $R_3$ und $R_4$ Wasserstoff,

Y $Na^{\oplus}$, $K^{\oplus}$ oder 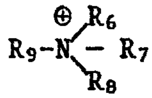 ,

$R_5$ $C_1$-$C_5$-Alkyl,

$R_6$, $R_7$, $R_8$ und $R_9$ unabhängig voneinander $C_1$-$C_4$-Alkyl und

n die Zahl 2 bedeuten.

4. Eine Verbindung gemäss Anspruch 3, worin $R_1$ Chlor, $R_2$, $R_3$ und $R_4$ Wasserstoff, Y $Na^{\oplus}$ oder $K^{\oplus}$, $R_5$ Methyl und n die Zahl 2 bedeuten.

5. Die Verbindung gemäss Anspruch 4 der Formel

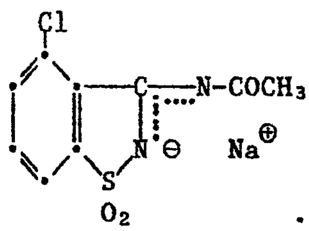
.

6. Die Verbindung gemäss Anspruch 3 der Formel

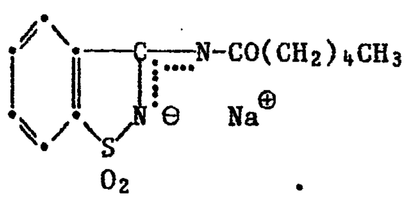
.

7. Die Verbindung gemäss Anspruch 2 der Formel

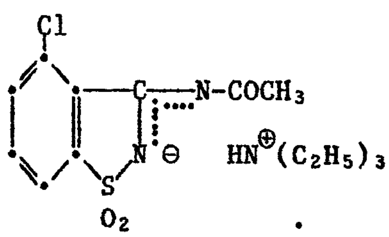
.

8. Die Verbindung gemäss Anspruch 3 der Formel

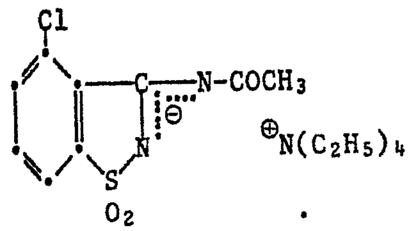

.

9. Die Verbindung gemäss Anspruch 3 der Formel

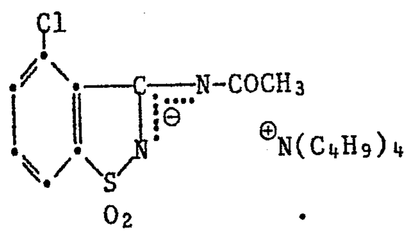

.

10. Die Verbindung gemäss Anspruch 3 der Formel

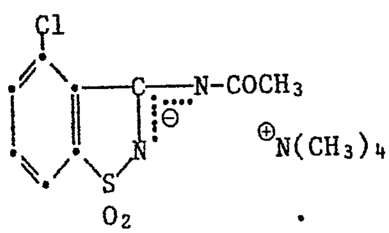

.

11. Die Verbindung gemäss Anspruch 4 der Formel

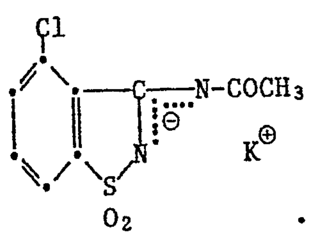

.

0207891

12. Die Verbindung gemäss Anspruch 4 der Formel

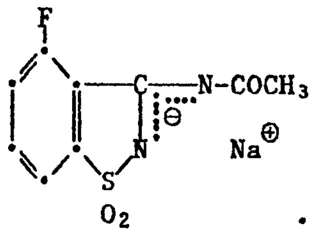

.

13. Die Verbindung gemäss Anspruch 1 der Formel

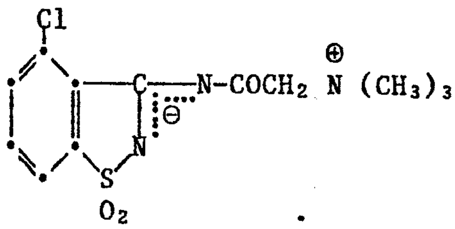

.

14. Verfahren zur Herstellung einer Verbindung der Formel I

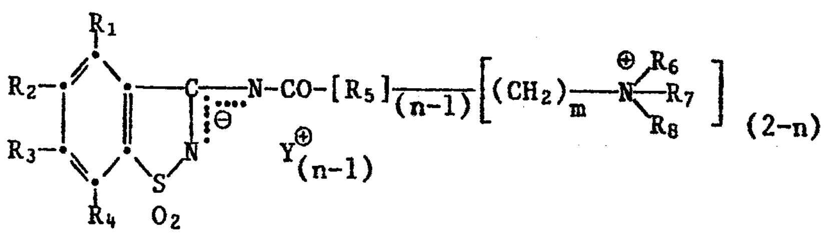

(I),

worin

$R_1$, $R_2$, $R_3$ und $R_4$ unabhängig voneinander Wasserstoff, Halogen, $C_1$-$C_5$-Alkyl, $C_1$-$C_5$-Alkoxy, Trifluormethyl, Amino oder Nitro,

Y $Na^{\oplus}$, $K^{\oplus}$ oder 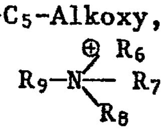 ,

$R_5$ gegebenenfalls substituiertes Alkyl, Cycloalkyl, Alkenyl, Alkinyl oder Phenyl,

$R_6$, $R_7$ und $R_8$ unabhängig voneinander Alkyl, Alkenyl oder Alkinyl,

$R_9$ Wasserstoff, Alkyl, Alkenyl oder Alkinyl,

n die Zahl 1 oder 2 und

m eine Zahl von 1 bis 10 bedeuten,

dadurch gekennzeichnet, dass man a) eine Verbindung der Formel

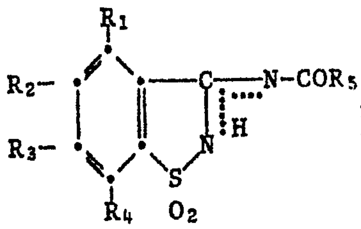 mit NaOH, KOH oder einer Verbindung der Formel 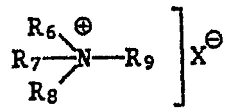

oder b) eine Verbndung der Formel

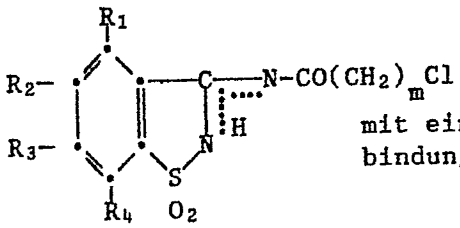 mit einer Verbindung der Formel 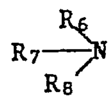 umsetzt,

worin in den Formeln $R_1$ - $R_9$ und m die angegebene Bedeutung haben und X ein Hydroxyl- oder Halogenanion ist.

15. Schädlingsbekämpfungsmittel, welches als aktive Komponente mindestens eine Verbindung der Formel I

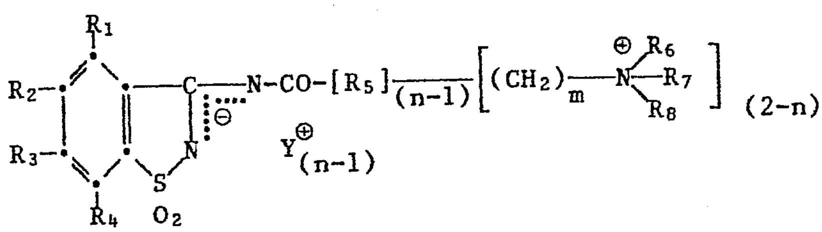 (I),

enthält, worin

$R_1$, $R_2$, $R_3$ und $R_4$ unabhängig voneinander Wasserstoff, Halogen, $C_1$-$C_5$-Alkyl, $C_1$-$C_5$-Alkoxy, Trifluormethyl, Amino oder Nitro,

Y $Na^{\oplus}$, $K^{\oplus}$ oder 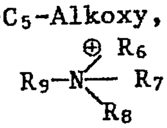 ,

$R_5$ gegebenenfalls substituiertes Alkyl, Cycloalkyl, Alkenyl, Alkinyl oder Phenyl,

$R_6$, $R_7$ und $R_8$ unabhängig voneinander Alkyl, Alkenyl oder Alkinyl,

$R_9$ Wasserstoff, Alkyl, Alkenyl oder Alkinyl,

n die Zahl 1 oder 2 und

m eine Zahl von 1 bis 10 bedeuten,

zusammen mit geeigneten Trägern und/oder Zuschlagstoffen.

16. Schädlingsbekämpfungsmittel gemäss Anspruch 15, welches als aktive Komponente mindestens eine Verbindung gemäss einem der Ansprüche 2 bis 13 enthält.

17. Verwendung einer Verbindung der Formel I

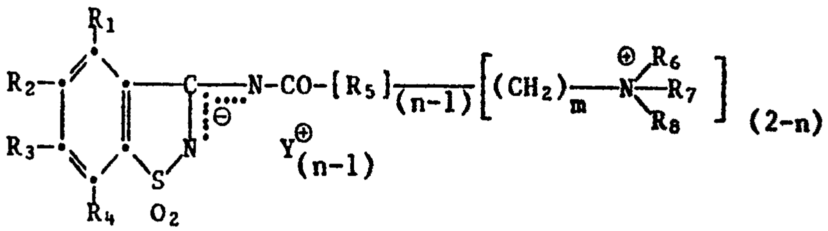

(I),

worin

$R_1$, $R_2$, $R_3$ und $R_4$ unabhängig voneinander Wasserstoff, Halogen, $C_1$-$C_5$-Alkyl, $C_1$-$C_5$-Alkoxy, Trifluormethyl, Amino oder Nitro,

Y $Na^{\oplus}$, $K^{\oplus}$ oder 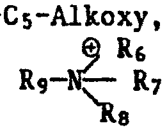 ,

$R_5$ gegebenenfalls substituiertes Alkyl, Cycloalkyl, Alkenyl, Alkinyl oder Phenyl,

$R_6$, $R_7$ und $R_8$ unabhängig voneinander Alkyl, Alkenyl oder Alkinyl,

$R_9$ Wasserstoff, Alkyl, Alkenyl oder Alkinyl,

n die Zahl 1 oder 2 und

m eine Zahl von 1 bis 10 bedeuten,

zur Bekämpfung von verschiedenartigen Schädlingen an Tieren, Pflanzen und im Boden.

18. Verwendung gemäss Anspruch 17 zur Bekämpfung von Insekten.

Europäisches Patentamt

# EUROPÄISCHER RECHERCHENBERICHT

0207891

Nummer der Anmeldung

EP 86 81 0256

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| X | EP-A-0 110 829 (CIBA GEIGY)<br>* Ansprüche 1-5,21-25 *<br>--- | 1-3,15 -18 | C 07 D 275/06<br>A 01 N 43/80 |
| A | EP-A-0 133 418 (CIBA GEIGY)<br>* Ansprüche *<br>----- | 1-3,15 -18 | |
| | | | RECHERCHIERTE SACHGEBIETE (Int. Cl.4) |
| | | | C 07 D 275/06 |

Der vorliegende Recherchenbericht wurde für alle Patentansprüche erstellt.

| Recherchenort | Abschlußdatum der Recherche | Prüfer |
|---|---|---|
| DEN HAAG | 26-09-1986 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument